# EUROPEAN PATENT APPLICATION

(11) **EP 2 722 006 A1**
(43) Date of publication of application: **23.04.2014**
(21) Application number: 13166731.3
(22) Date of filing: 07.05.2013
(51) Int. Cl.: A61B 8/08, A61B 8/00, A61B 7/02

(54) **Method and apparatus for generating volume image**

(30) Priority: 16.10.2012 KR 20120115034
(71) Applicant: Samsung Medison Co., Ltd., Gangwon-do 250-870 (KR)
(72) Inventor: Park, Sung-wook, Gangwon-do (KR); Lee, Jin-yong, Gangwon-do (KR)
(74) Representative: Lorenz, Markus

(57) **Abstract**

A method of generating a volume image. The method includes: acquiring a sound generated by a cyclically moving object; classifying the acquired sound into periods corresponding to cyclic motions of the cyclically moving object; generating sub-volume images corresponding to predetermined regions of a plurality of regions that a cyclically moving object has been divided into by transmitting ultrasound signals towards the cyclically moving object and receiving response signals reflected from the cyclically moving object, at every period; and generating a volume image of the object by connecting the generated sub-volume images to one other.

## Description

### CROSS-REFERENCE TO RELATED PATENT APPLICATION

This application claims the benefit of Korean Patent Application No. 10-2012-0115034, filed on October 16, 2012, in the Korean Intellectual Property Office, the disclosure of which is incorporated herein in its entirety by reference.

### BACKGROUND OF THE INVENTION

### 1. Field of the Invention

The present invention relates to a method and apparatus for generating a volume image of an object, and more particularly, to a method and apparatus for generating a volume image of an object by using a sound generated by the object.

### 2. Description of the Related Art

An ultrasound device is equipment for observing the inside structure of an organism. The ultrasound device is a noninvasive diagnostic device used to view structural details of internal tissue and a flow of a fluid inside the human body.

The ultrasound device acquires an image of a structure inside the human body by transmitting an ultrasound signal into the human body and towards an object and receiving a response signal reflected from the object.

The ultrasound device may generate a volume image that is a three-dimensional image of an object, and an examiner may three-dimensionally diagnose the object by using the volume image.

### SUMMARY OF THE INVENTION

The present invention provides a volume image generating method and apparatus for accurately generating a volume image of an object by using a sound generated by the object.

According to an aspect of the present invention, there is provided a method of generating a volume image, the method including: acquiring a sound generated by a cyclically moving object; classifying the acquired sound into periods corresponding to cyclic motions of the cyclically moving object; generating sub-volume images corresponding to predetermined regions of a plurality of regions that a cyclically moving object has been divided into by transmitting ultrasound signals towards the cyclically moving object and receiving response signals reflected from the cyclically moving object, at every period; and generating a volume image of the object by connecting the generated sub-volume images to one other.

The object may include a heart, and the classifying may include: determining a first sub-period and a second sub-period of the acquired sound respectively corresponding to a contraction period and a relaxation period of the heart; and classifying the acquired sound into the periods corresponding to the cyclic motions of the object based on the determined first sub-period and the determined second sub-period of the acquired sound.

Each of the periods corresponding to the cyclic motions of the object may include the determined first sub-period and the determined second sub-period.

The generating of the sub-volume images may include generating the sub-volume images at every determined first sub-period or determined second sub-period of the acquired sound.

The generating of the sub-volume images may include generating the sub-volume images at the same point in every period.

The predetermined regions respectively corresponding to the sub-volume images generated at every period may be different from each other.

The method may further include displaying the generated sub-volume images or the generated volume image.

The object may include the heart of a fetus.

A program, which when executed by a computer, performs the method, may be recorded in a non-transitory computer-readable storage medium.

According to another aspect of the present invention, there is provided an apparatus for generating a volume image, the apparatus including: a sound acquisition unit for acquiring a sound generated by a cyclically moving object; a sound classification unit for classifying the acquired sound into periods corresponding to cyclic motions of the cyclically moving object; and an image generator for generating sub-volume images corresponding to predetermined regions of a plurality of regions that a cyclically moving object has been divided into by transmitting ultrasound signals towards the cyclically moving object and receiving response signals reflected from the cyclically moving object, at every period, and generating a volume image of the object by connecting the generated sub-volume images to one other.

The object may include a heart, and the sound classification unit may determine a first sub-period and a second sub-period of the acquired sound respectively corresponding to a contraction period and a relaxation period of the heart and classifies the acquired sound into the periods corresponding to the cyclic motions of the object based on the determined first sub-period and the determined second sub-period of the acquired sound.

Each of the periods corresponding to the cyclic motions of the object may include the determined first sub-period and the determined second sub-period.

The image generator may generate the sub-volume images at every determined first sub-period or determined second sub-period of the acquired sound.

The image generator may generate the sub-volume images at the same point in every period.

The predetermined regions respectively corresponding to the sub-volume images generated at every period may be different from each other.

The apparatus may further include a display unit for displaying the generated sub-volume images or the generated volume image.

The object may include the heart of a fetus.

The apparatus may be included in an ultrasound diagnostic device.

### BRIEF DESCRIPTION OF THE DRAWINGS

The above and other features and advantages of the present invention will become more apparent by describing in detail exemplary embodiments thereof with reference to the attached drawings in which:
FIG. 1 is illustrates a conventional method of generating a volume image of a heart using an electrocardiogram (ECG);
FIG. 2 is a graph showing an ECG of a pregnant woman and an ECG of the fetus thereof;
FIG. 3 is a block diagram of an apparatus for generating a volume image, according to an embodiment of the present invention;
FIG. 4 is a graph showing a cardiac sound generated by a heart;
FIG. 5 illustrates a method of generating a volume image of an object by using a cardiac sound in an apparatus for generating a volume image, according to an embodiment of the present invention; and
FIG. 6 is a flowchart illustrating a method of generating a volume image, according to another embodiment of the present invention.

### DETAILED DESCRIPTION OF THE INVENTION

Advantages and features of the present invention and a method for achieving them will be clear with reference to the accompanying drawings, in which exemplary embodiments of the invention are shown. The invention may, however, be embodied in many different forms and should not be construed as being limited to the embodiments set forth herein; rather, these embodiments are provided so that this disclosure will be thorough and complete, and will fully convey the concept of the invention to one of ordinary skill in the art. Like reference numerals denote like elements throughout the specification.

The term '...unit' used in the embodiments indicates a component including software or hardware, such as a Field Programmable Gate Array (FPGA) or an Application-Specific Integrated Circuit (ASIC), and the '...unit' performs certain roles. However, the '...unit' is not limited to software or hardware. The '...unit' may be configured to be included in an addressable storage medium or to reproduce one or more processors. Therefore, for example, the '...unit' includes components, such as software components, object-oriented software components, class components, and task components, processes, functions, attributes, procedures, subroutines, segments of program code, drivers, firmware, microcode, circuits, data, a database, data structures, tables, arrays, and variables. A function provided inside components and '...units' may combine them into a smaller number of components and '...units' or further divide them into additional components and '...units'.

In the specification, 'object' indicates various organs or a specific part of an animal or the human body for which ultrasound images are to be acquired. The object may include a heart, and particularly, include the heart of a fetus.

One of methods of acquiring a volume image of an object is to generate a volume image of the object at once by transmitting ultrasound signals towards the entire object and receiving response signals reflected from the object. However, the volume image generated by the method has a disadvantage in that spatial and temporal resolutions are low.

As a method for increasing spatial and temporal resolutions of a volume image, a volume image of an object may be generated by dividing the object into a plurality of regions, generating sub-volume images of the plurality of regions, and connecting the generated sub-volume images. However, even by this method, it is difficult to generate an accurate volume image of a cyclically moving object, such as the heart of the human body, because an outward shape of the cyclically moving object changes over time, resulting in mismatches between sub-volume images of the object. To solve this, connectivity between the sub-volume images should be increased by generating a sub-volume image at every specific time point in periods of motion of the object.

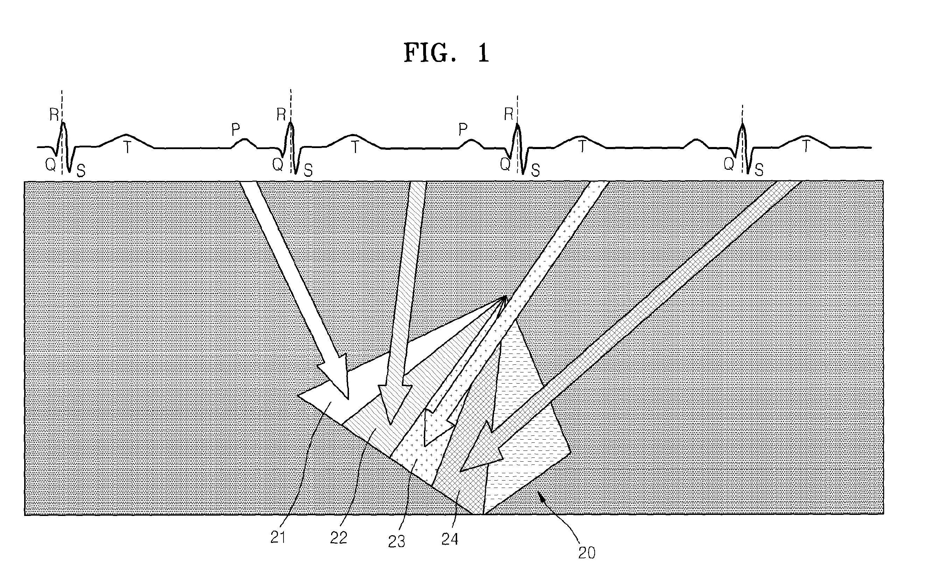

FIG. 1 is illustrates a conventional method of generating a volume image of a heart using an electrocardiogram (ECG).

Referring to FIG. 1, a heart is divided into four regions 21, 22, 23, and 24, and a cardiac ECG corresponds to four periods of motion of the heart. Conventionally, connectivity between sub-volume images has been increased by acquiring sub-volume images of an object at every specific point in a cardiac cycle time period represented in the cardiac ECG. However, this conventional method has low accuracy when a volume image of the heart of a fetus is generated, because it is difficult to accurately identify an ECG of the fetus due to an influence of an ECG of a pregnant woman carrying the fetus on the ECG of the fetus.

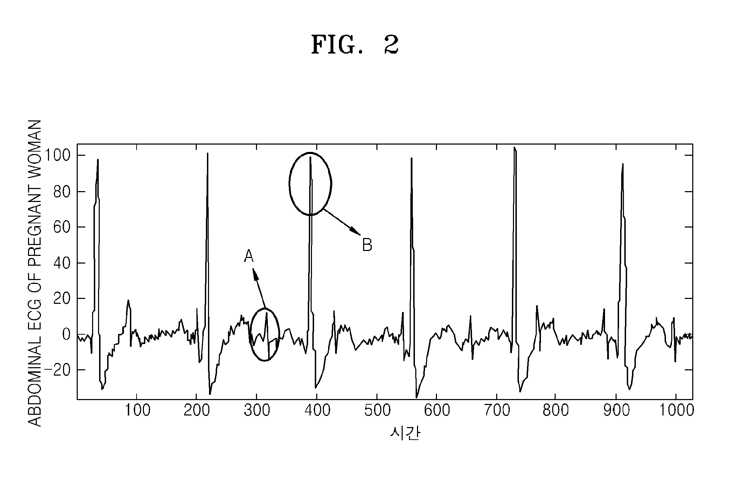

FIG. 2 is a graph showing an ECG of a pregnant woman and an ECG of the fetus thereof.

In general, a signal recorded with respect to the abdomen of the pregnant woman has a complicated form due to an ECG of the pregnant woman and random noise. In FIG. 2, a cyclically appearing main peak B appears due to cardiac activity of the pregnant woman, and a relatively small peak A having a relatively short period appears due to cardiac activity of the fetus. If each cyclic peak in the ECG of the fetus is influenced by peaks in the ECG of the pregnant woman, it is difficult to identify the cyclic peak in the ECG of the fetus. Accordingly, an accurate time point for generating sub-volume images of the heart of the fetus may be unable to be determined.

The present invention may accurately generate a volume image of an object of a fetus by using a sound generated by the object of the fetus.

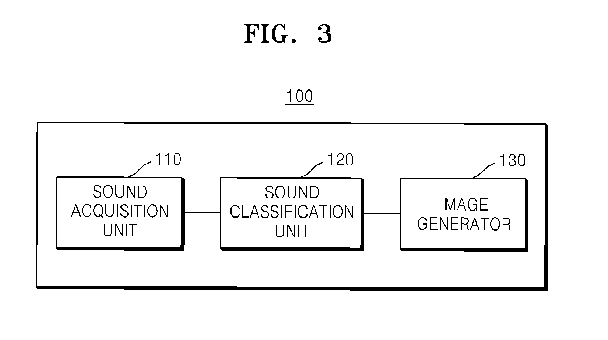

FIG. 3 is a block diagram of an apparatus 100 for generating a volume image, according to an embodiment of the present invention.

Referring to FIG. 3, the apparatus 100 may include a sound acquisition unit 110, a sound classification unit 120, and an image generator 130. The sound acquisition unit 110, the sound classification unit 120, or the image generator 130 may be formed by a microprocessor, and the apparatus 100 may be included in an ultrasound diagnostic device.

The sound acquisition unit 110 may acquire a sound generated by a cyclically moving object. The sound generated by the cyclically moving object may have periodicity. The sound acquisition unit 110 may include well-known devices for acquiring the sound generated by a cyclically moving object of the human body, such as a stethoscope, a microphone, and the like.

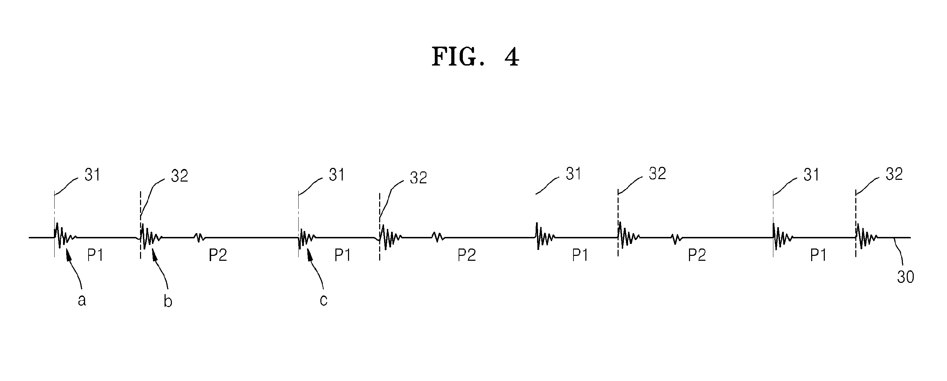

The sound classification unit 120 may classify the sound acquired by the sound acquisition unit 110 into periods corresponding to cyclic motions of the cyclically moving object. This will now be described with reference to FIG. 4.

FIG. 4 is a graph showing a cardiac sound 30 generated by a heart.

Referring to FIG. 4, a first vibration a having a high amplitude, a second vibration b having a high amplitude, and a third vibration c having a low amplitude form one period. The cardiac sound 30 shown in FIG. 4 is cyclically classified by four lines 31.

According to an ECG, the heart contracts after an R wave is generated, and relaxes after a T wave is generated. According to the cardiac sound 30, the heart contracts after the first vibration a and relaxes after the second vibration b. That is, in each period of the cardiac sound 30 shown in FIG. 4, P1 denotes a contraction period, and P2 denotes a relaxation period.

The sound classification unit 120 may determine a first sub-period P1 and a second sub-period P2 respectively corresponding to a contraction period and a relaxation period of the heart and classify the cardiac sound 30 into periods corresponding to cyclic motions of the cyclically moving object. In detail, the sound classification unit 120 may classify the cardiac sound 30 so that each period of the cardiac sound 30 includes the first sub-period P1 and the second sub-period P2.

The image generator 130 may transmit ultrasound signals to the cyclically moving object, which has been divided into a plurality of regions, and receive response signals reflected from the cyclically moving object, at every period of the sound generated by the cyclically moving object. The image generator 130 may include a probe for transmitting ultrasound signals towards the cyclically moving object and receiving response signals reflected from the cyclically moving object.

The image generator 130 may generate sub-volume images corresponding to predetermined regions of the plurality of regions of the cyclically moving object based on the received response signals and generate a volume image of the cyclically moving object by connecting the generated sub-volume images to one other.

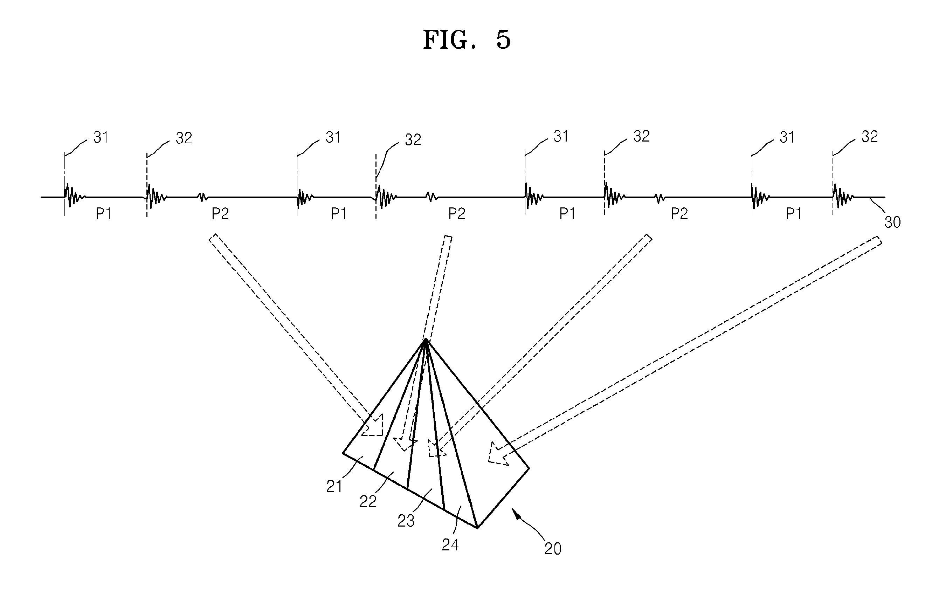

An operation of generating sub-volume images in the image generator 130 will now be described in detail with reference to FIG. 5.

FIG. 5 illustrates a method of generating a volume image of the cyclically moving object by using the cardiac sound 30 in the apparatus 100 for generating a volume image, according to an embodiment of the present invention.

Referring to FIG. 5, the image generator 130 may generate sub-volume images of the object at every P2 period in periods of the cardiac sound 30. In addition, the image generator 130 may generate sub-volume images of the cyclically moving object at every P1 period. According to this, connectivity between the sub-volume images generated by the image generator 130 may be increased. The image generator 130 may generate sub-volume images of the cyclically moving object at the same point in every period of the cardiac sound 30.

Each of the sub-volume images generated at every period by the image generator 130 corresponds to a different region of the cyclically moving object. That is, referring to FIG. 5, the image generator 130 generates a first sub-volume image 21, a second sub-volume image 22, a third sub-volume image 23, and a fourth sub-volume image 24 corresponding to different regions of the cyclically moving object at a first P2 period, a second P2 period, a third P2 period, and a fourth P2 period, respectively.

Although not shown in FIG. 3, the apparatus 100 may further include a display unit for displaying sub-volume images or a volume image generated by the image generator 130.

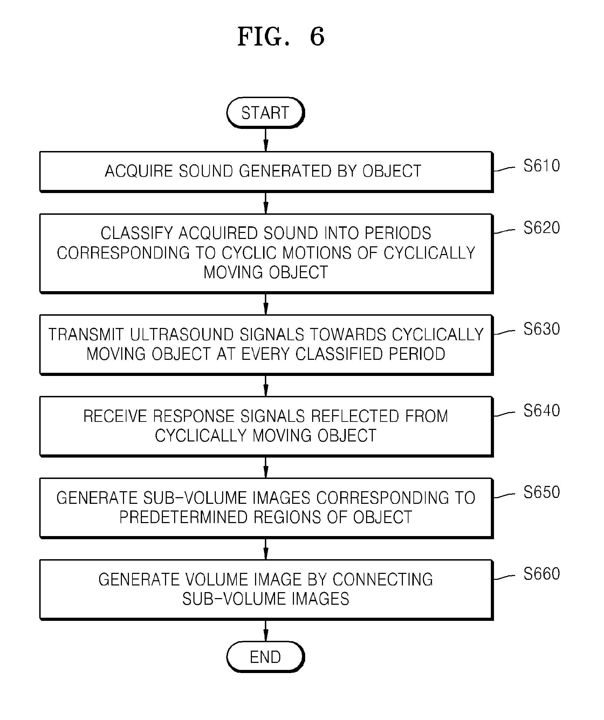

FIG. 6 is a flowchart illustrating a method of generating a volume image, according to another embodiment of the present invention. Referring to FIG. 6, the method includes operations processed by the apparatus 100 of FIG. 3. Thus, although omitted hereinafter, the description related to the apparatus 100 of FIG. 3 may also be applied to the method of FIG. 6.

In operation S61 0, the apparatus 100 acquires a sound generated by a cyclically moving object. The cyclically moving object may include the heart of a fetus.

In operation S620, the apparatus 100 classifies the sound into periods corresponding to cyclic motions of the cyclically moving object. When the cyclically moving object includes the heart, the apparatus 100 may classify the sound so that each of the periods includes a contraction period and a relaxation period of the heart.

In operation S630, the apparatus 100 transmits ultrasound signals to the cyclically moving object at every period of the sound.

In operation S640, the apparatus 100 receives response signals reflected from the cyclically moving object.

In operation S650, the apparatus 100 generates sub-volume images corresponding to predetermined regions that a cyclically moving object has been divided into, based on the received response signals.

In operation S660, the apparatus 100 generates a volume image of the cyclically moving object by connecting the generated sub-volume images to one other.

Although it has been described in the specification that a 'heart' is an example of the cyclically moving object, it will be obvious to one of ordinary skill in the art that the technical feature of the present invention is also applicable to other objects, which generate a cyclic sound by cyclically moving.

The embodiments of the present invention can be written as computer programs and can be implemented in general-use digital computers that execute the programs using a computer-readable storage medium. Examples of the computer-readable storage medium include storage media such as magnetic storage media (e.g., ROM, floppy disks, hard disks, etc.), optical recording media (e.g., CD-ROMs, or DVDs), and carrier waves (e.g., transmission through the Internet).

While embodiments of the present invention have been described with reference to the accompanying drawings, it will be understood by one of ordinary skill in the art that various changes in form and details may be made therein without changing the technical spirit and mandatory features of the present invention. Therefore, the embodiments should be understood in the illustrative sense only and not for the purpose of limitation in all aspects.

## Claims

1. A method of generating a volume image, the method comprising:
acquiring a sound generated by a cyclically moving object;
classifying the acquired sound into periods corresponding to cyclic motions of the cyclically moving object;
generating sub-volume images corresponding to predetermined regions of a plurality of regions that a cyclically moving object has been divided into by transmitting ultrasound signals towards the cyclically moving object and receiving response signals reflected from the cyclically moving object, at every period; and
generating a volume image of the object by connecting the generated sub-volume images to one other.

2. The method of claim 1, wherein the object includes a heart, and
the classifying comprises:
determining a first sub-period and a second sub-period of the acquired sound respectively corresponding to a contraction period and a relaxation period of the heart; and
classifying the acquired sound into the periods corresponding to the cyclic motions of the object based on the determined first sub-period and the determined second sub-period of the acquired sound.

3. The method of claim 2, wherein each of the periods corresponding to the cyclic motions of the object includes the determined first sub-period and the determined second sub-period.

4. The method of claim 2, wherein the generating of the sub-volume images comprises generating the sub-volume images at every determined first sub-period or determined second sub-period of the acquired sound.

5. The method of claim 1, wherein the generating of the sub-volume images comprises generating the sub-volume images at the same point in every period.

6. The method of claim 1, wherein the predetermined regions respectively corresponding to the sub-volume images generated at every period are different from each other.

7. The method of claim 1, further comprising displaying the generated sub-volume images or the generated volume image.

8. The method of claim 1, wherein the object includes the heart of a fetus.

9. A non-transitory computer-readable storage medium recording therein a program, which when executed by a computer, performs the method of any one of claims 1 through 8.

10. An apparatus for generating a volume image, the apparatus comprising:
a sound acquisition unit for acquiring a sound generated by a cyclically moving object;
a sound classification unit for classifying the acquired sound into periods corresponding to cyclic motions of the cyclically moving object; and
an image generator for generating sub-volume images corresponding to predetermined regions of a plurality of regions that a cyclically moving object has been divided into by transmitting ultrasound signals towards the cyclically moving object and receiving response signals reflected from the cyclically moving object, at every period, and generating a volume image of the object by connecting the generated sub-volume images to one other.

11. The apparatus of claim 10, wherein the object includes a heart, and the sound classification unit determines a first sub-period and a second sub-period of the acquired sound respectively corresponding to a contraction period and a relaxation period of the heart and classifies the acquired sound into the periods corresponding to the cyclic motions of the object based on the determined first sub-period and the determined second sub-period of the acquired sound.

12. The apparatus of claim 11, wherein each of the periods corresponding to the cyclic motions of the object includes the determined first sub-period and the determined second sub-period.

13. The apparatus of claim 11, wherein the image generator generates the sub-volume images at every determined first sub-period or determined second sub-period of the acquired sound.

14. The apparatus of claim 10, wherein the image generator generates the sub-volume images at the same point in every period.

15. The apparatus of claim 10, wherein the predetermined regions respectively corresponding to the sub-volume images generated at every period are different from each other.
